# EUROPEAN PATENT APPLICATION

(11) **EP 2 407 136 A1**
(43) Date of publication of application: **18.01.2012**
(21) Application number: 10750917.6
(22) Date of filing: 12.03.2010
(51) Int. Cl.: A61F 13/30, A61F 13/32

(54) **TAMPON APPLICATOR**

(30) Priority: 13.03.2009 JP 2009061700
(71) Applicant: Unicharm Corporation, Ehime 799-0111 (JP)
(72) Inventor: WATANABE, Hitoshi, Kanonji-shi Kagawa 769-1602 (JP)
(74) Representative: Eke, Philippa Dianne
(86) International application number: PCT/JP2010/054174
(87) International publication number: WO 2010/104166

(57) **Abstract**

A tampon applicator 100 according to the present invention includes an outer tube 110 and an inner tube 120. The outer tube 110 includes an outer-tube tip end portion 111 having a locking portion 213 and an outer-tube rear end portion 112 having a protrusion 200. The protrusion 200 is capable of passing by the locking portion 213 from the outer-tube rear end portion 112 side to the outer-tube tip end portion 111 side. When the protrusion 200 is positioned on the outer-tube tip end portion 111 side of the locking portion 213, the locking portion 213 locks the protrusion 200 to prevent the inner tube 120 from returning to the outer-tube rear end portion 112 side of the locking portion 200.

## Description

### [Technical Field]

The present disclosure relates to a tampon applicator including an outer tube in which a tampon is ejectably placed and a pushing body which pushes the tampon in the outer tube toward an outside of the outer tube.

### [Background Art]

A tampon applicator for a sanitary tampon is placed with a tampon configured to absorb body fluid such as menstrual fluid, and is used to insert the tampon into the vagina of a user. In general, a tampon applicator widely adopts a sliding-type application system in which any one of a pair of hollow tube bodies moves slidingly with respect to the other.

Specifically, the tampon applicator includes a hollow outer tube in which the tampon is ejectably placed, and an inner tube (a pushing body) which pushes the tampon toward an outside of the outer tube by slidingly moving in the outer tube.

The outer tube has a main body portion in which the tampon is placed, an outer-tube tip end portion which is inserted into the vagina of a user, and an outer-tube rear end portion which is positioned opposite to the outer-tube tip end portion. The outer-tube tip end portion has an opening from which the tampon is ejected.

The inner tube has an inner-tube tip end portion (a pushing tip end portion) which pushes the tampon toward the outside of the outer tube and an inner tube rear end (a pushing rear end portion) which is positioned opposite to the inner-tube tip end portion and is pushed by a user toward the outer-tube tip end portion.

The tampon applicator is inserted from the outer-tube tip end portion into the vagina of the user. The user pushes the inner tube rear end toward the outer-tube tip end portion. With this movement, the tampon placed in the outer tube is pushed by the inner tube (the inner-tube tip end portion) so that the tampon is inserted into the vagina of the user.

### [Prior Art Document]

### [Patent Document]

### [Patent Document 1]

Japanese Patent Application Publication No.Hei. 3-38854

### [Summary of Invention]

In some situations such as when a sanitary container or the like is not near a user of a sanitary tampon, the user has to bring back a tampon applicator having no tampon after use.

In such a case, the above-described conventional tampon applicator has the following problem. That is, it is extremely hard to see from an outside of the tampon applicator whether the tampon is placed inside the tampon applicator. This causes the problem that, although a tampon is not placed inside the tampon applicator, a user accidentally uses the sanitary tampon in which the tampon is not placed in the tampon applicator.

Accordingly, an object of the invention is to provide a tampon applicator capable of securely preventing a user from accidentally using a sanitary tampon in which a tampon is not placed in a tampon applicator.

To solve the above problem, the present invention has the following aspect. The aspect of the present invention is summarized as a tampon applicator (e.g., a tampon applicator 100) including: an outer tube (outer tube 110) in which a tampon (tampon 10) is ejectably placed; and a pushing body (inner tube 120) which pushes the tampon toward the outside of the outer tube by slidingly moving in the outer tube. In the tampon applicator, the outer tube includes: an outer-tube tip end portion (outer-tube tip end portion 111), on which an opening (opening 111A) to eject the tampon therefrom is formed, and which is inserted into a vagina of a user; and an outer-tube rear end portion (outer-tube rear end portion 112) positioned opposite to the outer-tube tip end portion. The pushing body includes: a pushing tip end portion (pushing tip end portion 121) which pushes the tampon toward the outside of the outer tube; a pushing rear end portion (pushing rear end portion 122) positioned opposite to the pushing tip end portion; and a protrusion (e.g., a protrusion 200) provided in the vicinity of the pushing rear end portion and protruding from the outer circumference of the pushing body. The outer tube is provided with a locking portion (locking portion 213) which locks the protrusion when the pushing body is inserted in the outer tube. Along with a sliding movement of the pushing body in the outer tube, the protrusion is capable of passing by the locking portion from the outer-tube rear end portion side to the outer-tube tip end portion side. When the protrusion is positioned on the outer-tube tip end portion side of the locking portion, the locking portion locks the protrusion to prevent the pushing body from returning to the outer-tube rear end portion side of the locking portion.

### [Brief Description of Drawings]

[Fig. 1] Fig. 1 is a perspective view of a sanitary tampon 1 according to a first embodiment;
[Fig. 2] Fig. 2 is a side view of the sanitary tampon 1 according to the first embodiment;
[Fig. 3] Fig. 3 is a partially-exploded side view of an outer tube 110 according to the first embodiment;
[Fig. 4] Figs. 4 (a) and 4 (b) are views showing an inner tube 120 according to the first embodiment;
[Fig. 5] Figs. 5 (a) and 5 (b) are views showing a protrusion 200 according to the first embodiment;
[Fig. 6] Figs. 6 (a) and 6 (b) are views showing a protrusion 210 according to Variation 1;
[Fig. 7] Figs. 7 (a) and 7 (b) are views showing a protrusion 220 according to Variation 2;
[Fig. 8] Figs. 8 (a) and 8 (b) are views showing a protrusion 230 according to Variation 3;
[Fig. 9] Figs. 9 (a) and 9 (b) are views showing a protrusion 240 according to Modification 1;
[Fig. 10] Figs. 10 (a) and 10 (b) are views showing a protrusion 250 according to Modification 2;
[Fig. 11] Figs. 11 (a) and 11 (b) are views showing a protrusion 260 according to Modification 3;
[Fig. 12] Fig. 12 is a perspective view (Part 1) of a sanitary tampon 1A according to a second embodiment;
[Fig. 13] Fig. 13 is a side view of the sanitary tampon 1A according to the second embodiment;
[Fig. 14] Fig. 14 is a partially-exploded side view of an outer tube 110 according to the second embodiment;
[Fig. 15] Fig. 15 is a view of an inner tube 120 according to the second embodiment;
[Fig. 16] Fig. 16 is a perspective view (Part 2) of the sanitary tampon 1A according to the second embodiment; and
[Fig. 17] Fig. 17 is a perspective view of a sanitary tampon 1 according to another embodiment.

### [Description of Embodiments]

A tampon applicator according to the invention is described below by referring to the drawings. Specifically, first, second, and other embodiments are described.

Note that, in the following description of the drawings, same or similar reference signs denote same or similar elements and portions. In addition, it should be noted that the drawings are schematic and ratios of dimensions and the like are different from actual ones.

Therefore, specific dimensions and the like should be determined in consideration of the following description. Moreover, the drawings also include portions having different dimensional relationships and ratios from each other.

### [First Embodiment]

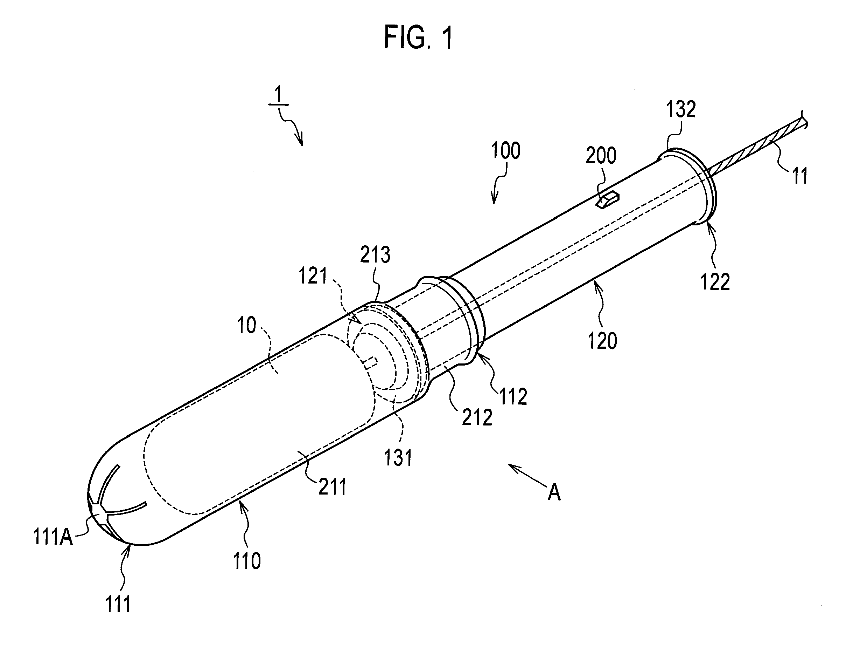

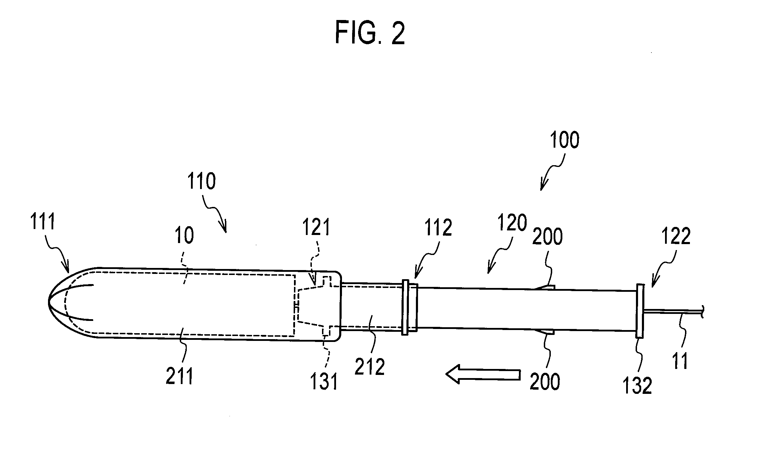

The structure of a sanitary tampon 1 according to a first embodiment is described by referring to the drawings. Fig. 1 is a perspective view showing the sanitary tampon 1 according to the first embodiment. Fig. 2 is a side view (seen from the direction shown by arrow A in Fig. 1) showing the sanitary tampon 1 according to the first embodiment.

As shown in Figs. 1 and 2, the sanitary tampon 1 has a tampon 10 (an absorber) which absorbs body fluid such as menstrual fluid and a tampon applicator 100 for inserting the tampon 10 into a vagina of a user.

The tampon 10 is placed, in a compressed state, in an outer tube 110, to be described later, of the tampon applicator 100. The tampon 10 includes an absorber (not shown) having absorbable fibers and a covering material (not shown) covering the absorber. A cord-like body 11 is connected with the tampon 10 and is drawn to eject the tampon 10 from the vagina of the user.

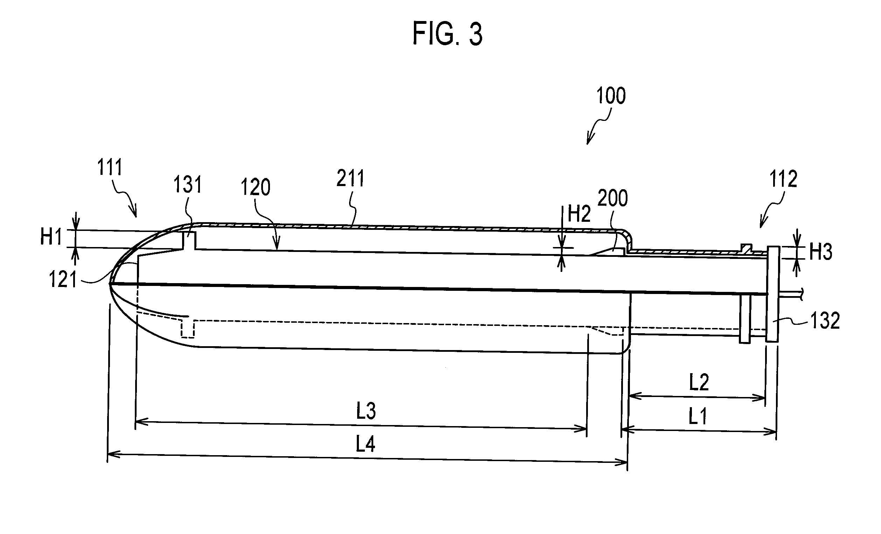

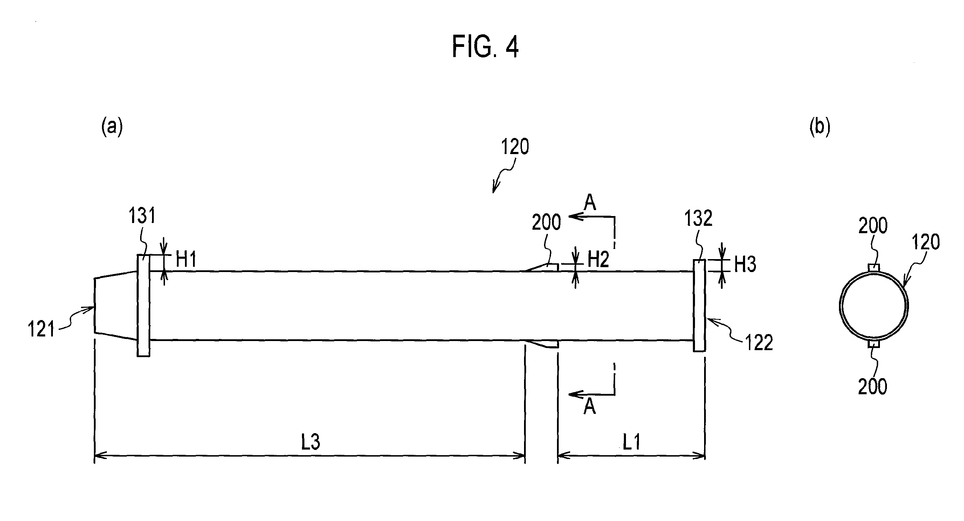

Next, the structure of the above-described tampon applicator 100 is described by referring to Figs. 1 to 4. Fig. 3 is a partially-exploded side view of the outer tube 110 according to the first embodiment. Fig. 4 (a) is a side view of an inner tube 120 according to the first embodiment. Fig. 4 (b) is a front view (a cross-sectional view taken along the A-A line in Fig. 4 (a)) of the inner tube 120 according to the first embodiment.

As shown in Figs. 1 to 4, the tampon applicator 100 includes a hollow outer tube 110 in which the tampon 10 is ejectably placed, and a hollow inner tube 120 (pushing body) into which the above-described cord-like body 11 is inserted and which pushes the tampon 10 toward the outside of the outer tube 110 by slidingly moving in the outer tube 110 .

It should be noted that the outer tube 110 and the inner tube 120 are formed of the same material such as, for example, polyethylene (PE), polyethylene terephthalate (PET), polypropylene (PP), a laminated nonwoven fiber, or paper.

The outer tube 110 has an outer-tube tip end portion 111 and an outer-tube rear end portion 112. The outer-tube tip end portion 111 has an opening 111A to eject the tampon 10 therefrom, and is inserted into the vagina of the user. The opening 111A gradually tapers toward the outer-tube tip end portion 111 and opens by being pushed by the tampon 10 from the inside of the outer tube 110. On the other hand, the outer-tube rear end portion 112 is positioned opposite to the outer-tube tip end portion 111, and the inner tube 120 is always inserted thereinto.

The outer tube 110 includes a main body portion 211 and a grip portion 212 (a small diameter portion). The main body portion 211 has a predetermined diameter. The diameter of the main body portion 211 is constant. On the other hand, the grip portion 212 is provided closer to the outer-tube rear end portion 112 than the main body portion 211. The diameter of the grip portion 212 is smaller than that of the main body portion 211, and is constant. The main body portion 211 and the grip portion 212 are continuous at a substantially right angle when seen from the side of the outer tube 110.

In addition, the outer tube 110 has a locking portion 213 which locks a later-described protrusion 200 formed in the outer tube 120 when the inner tube 120 is inserted in the outer tube 110. When the protrusion 200 is positioned on the outer-tube tip end portion 111 side of the locking portion 213, the locking portion 213 locks the protrusion 200 to prevent the inner tube 120 from returning to the outer-tube rear end portion 112 side of the locking portion 213.

The locking portion 213 is provided between the main body portion 211 and the grip portion 212. In other words, the locking portion 213 is in a boundary where the main body portion 211 and the grip portion 212 are continuous.

The inner tube 120 has substantially the same diameter as the grip portion 212. The "substantially the same" diameter includes a diameter slightly smaller than that of the grip portion 212. The inner tube 120 has a pushing tip end portion 121 and a pushing rear end portion 122.

The pushing tip end portion 121 pushes the tampon 10 toward the outside of the outer tube 110. On the other hand, the pushing rear end portion 122 is positioned opposite to the pushing tip end portion 121 and is pushed toward the outer-tube tip end portion 111 by the user. The inner tube 120 has a tip-end-side projection 131, a rear-end-side projection 132, and the protrusion 200.

The tip-end-side projection 131 is provided closer to the pushing tip end portion 121 than the protrusion 200 is and projects from the outer circumference of the inner tube 120 outward beyond the diameter of the grip portion 212. It is preferable that the tip-end-side projection 131 be formed within 30 mm towards the pushing tip end portion 122 from the pushing tip end portion 121. It is also preferable that a protruding height H1 of the tip-end-side projection 131 from the outer circumference of the inner tube 120 be larger than a protruding height H2 of the protrusion 200 from the outer circumference of the inner tube 120.

The rear-end-side projection 132 is provided closer to the pushing rear end portion side 122 than the protrusion 200 is and projects from the outer circumference of the inner tube 120 outward beyond the diameter of the grip portion 212. It is preferable that the rear-end-side projection 132 be formed within 30 mm towards the pushing tip end portion 121 from the pushing rear end portion 122. It is also preferable that a protruding height H3 of the rear-end-side projection 132 from the outer circumference of the inner tube 120 be larger than the protruding height H2 of the protrusion 200 from the outer circumference of the inner tube 120.

The protrusion 200 is provided in the vicinity of the pushing rear end portion 122 and projects from the outer circumference of the inner tube 120. The protrusion 200 includes two protrusions 200 placed facing each other across an axis of the inner tube 120.

A length L1 from the protrusion 200 to the pushing rear end portion 122 in a longitudinal direction of the inner tube 120 is larger than a length L2 of the grip portion 212 in the longitudinal direction of the outer tube 110. In addition, a length L3 from the protrusion 200 to the pushing tip end portion 121 in the longitudinal direction of the inner tube 120 is smaller than a length L4 of the main body portion 211 in the longitudinal direction of the outer tube 110.

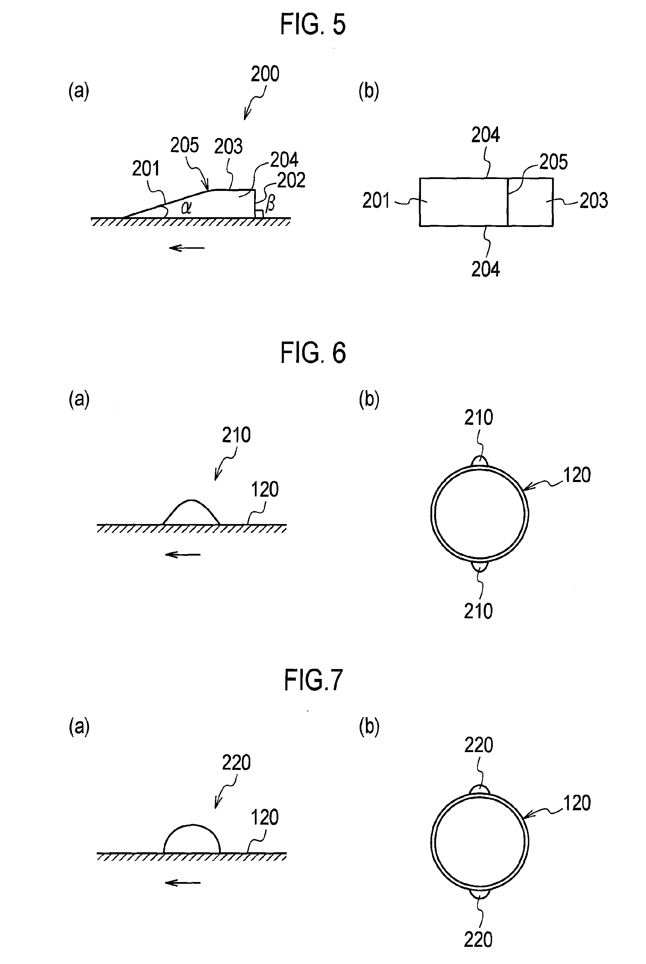

Next, a shape of the above-described protrusion 200 is described by referring to Figs. 5 (a) and 5 (b). Fig. 5 (a) is a side view of the protrusion 200 according to the first embodiment. Fig. 5 (b) is a top view of the protrusion 200 according to the first embodiment.

The protrusion 200, as shown in Figs. 5 (a) and 5 (b), along with the sliding movement of the inner tube 120 in the outer tube 110, can pass by the locking portion 213 from the outer-tube rear end portion 112 side to the outer-tube tip end portion 111 side. The protrusion 200 has a substantially trapezoidal shape when seen from the side thereof. Specifically, the protrusion 200 includes a front face 201 (a front portion), a rear face 202 (a rear portion), a top face 203, and a pair of side faces 204.

The front face 201 is positioned facing toward the pushing tip end portion 121. The front face 201 is inclined with respect to the outer circumference of the inner tube 120, and extends toward the pushing rear end portion 122 while gradually rising outward from the outer circumference of the inner tube 120. It is preferable that an angle α, which is an inner angle of the front face 201 and the outer circumference of the inner tube 120 be 10° to 90°, or, more preferably, 30° to 60°.

The rear face 202 is positioned opposite to the front face 201 and closer to the pushing rear end portion 122 than the front face 201. The rear face 202 is substantially perpendicular to the outer circumference of the inner tube 120. It is preferable that an angle β, which is an outer angle of the rear face 202 and the outer circumference of the inner tube 120, be 30° to 120°, or, more preferably, 90° to 120°.

When the protrusion 200 is seen from the side thereof, the top face 203 is continuous with the front face 201 and the rear face 202, and is substantially parallel with the outer circumference of the inner tube 120. It is preferable that an angular portion 205 at which the top face 203 and the front face 201 are continuous be formed in an arcuate shape (an R-letter shape). When the protrusion 200 is seen from the top face thereof, the paired side faces 204 are continuous with the front face 201 and the rear face 202, and face each other substantially in parallel.

In the above-described first embodiment, along with the sliding movement of the inner tube 120 in the outer tube 110, the protrusion 200 can pass by the locking portion 213 from the outer-tube rear end portion 112 side to the outer-tube tip end portion 111 side. With this structure, the protrusion 200 passes the grip portion 212 without being caught on the grip portion 212 and moves to the main body portion 211. Thus, when the tampon 10 is pushed toward the outside of the outer tube 110, the protrusion 200 does not become an obstacle for the inner tube 120.

In addition, when the protrusion 200 is positioned on the outer-tube tip end portion 111 side of the locking portion 213, the locking portion 213 locks the protrusion 200 to prevent the inner tube 120 from returning to the outer-tube rear end portion 122 side of the locking portion 213. In other words, after the inner tube 120 pushes the tampon 10 toward the outside of the outer tube 110, the locking portion 213 locks the protrusion 200, preventing the inner tube 120 from returning to the state before the sanitary tampon 1 is used. With this structure, the user can see from the outside of the sanitary tampon 1 if the tampon 10 is placed inside the tampon applicator 100. This surely prevents the user from accidentally using the sanitary tampon 1 in which the tampon 10 is not placed in the tampon applicator 100.

In the embodiment, the locking portion 213 is provided between the main body portion 211 and the grip portion 212. In other words, the locking portion 213 is in a boundary where the main body portion 211 and the grip portion 212 are continuous. In general, the grip portion 212 has a smaller diameter than the main body portion 211 so that the user can easily handle the sanitary tampon 1. For this reason, there is no need to include the locking portion 213 in the outer tube 110. Accordingly, the manufacturing cost of the sanitary tampon 1 can be reduced.

In the embodiment, the front face 201 is inclined with respect to the outer circumference of the inner tube 120, and extends toward the pushing rear end portion 122 while gradually rising outward from the outer circumference of the inner tube 120. It is preferable that the angle α between the front face 201 and the outer circumference of the inner tube 120 be 10° to 90°. This structure allows the protrusion 200 to easily pass inside the grip portion 212. Thus, the protrusion 200 can be prevented from being caught on the grip portion 212 when the inner tube 120 pushes the tampon 10 toward the outside of the outer tube 110.

If the angle α is smaller than 10°, the protrusion 200 cannot have a sufficient height, causing the protrusion 200 having passed the locking portion 213 to easily return toward the outer-tube rear end portion 112. Furthermore, when it is attempted to give the protrusion 200 a height needed for the locking portion 213 to lock the protrusion 200, the length of the protrusion 200 in the longitudinal direction of the inner tube 120 becomes longer. As a result, it becomes difficult for the protrusion 200 to pass inside the grip portion 212. On the other hand, if the angle α is larger than 90°, it becomes difficult for the protrusion 200 to get into the grip portion 212.

In the embodiment, the rear face 202 is substantially perpendicular to the outer circumference of the inner tube 120. It is preferable that the angle β between the rear face 202 and the outer circumference of the inner tube 120 be 30° to 120°. With this structure, the protrusion 200 can pass the grip portion 212, and the locking portion 213 can securely lock the rear face 202. Accordingly, it is difficult for the inner tube 120 to return to the state before the sanitary tampon 1 is used, after the locking portion 213 locks the protrusion 200.

If the angle β is smaller than 30°, when it is attempted to give the protrusion 200 a height needed for the locking portion 213 to lock the protrusion 200, the length of the protrusion 200 in the longitudinal direction of the inner tube 120 becomes longer. As a result, it becomes difficult for the protrusion 200 to pass inside the grip portion 212. On the other hand, if the inclination angle β is larger than 120°, it becomes easy for the inner tube 120 to return to the state before the sanitary tampon 1 is used.

In the embodiment, the length L1 from the protrusion 200 to the pushing rear end portion 122 in the longitudinal direction of the inner tube 120 is larger than the length L2 of the grip portion 212 in the longitudinal direction of the outer tube 110. If the length L1 is smaller than the length L2, the protrusion 200 cannot pass the grip portion 212, which prevents the protrusion 200 from reaching the locking portion 213. As a result, it becomes easy for the inner tube 120 to return to the state before the sanitary tampon 1 is used.

In the embodiment, the length L3 from the protrusion 200 to the pushing tip end portion 121 in the longitudinal direction of the inner tube 120 is smaller than the length L4 of the main body portion 211 in the longitudinal direction of the outer tube 110. If the length L3 is larger than the length L4, the inner tube 120 (the pushing tip end portion 121) comes out from the outer tube 110 when the protrusion 200 passes by the locking portion 213 from the outer-tube rear end portion 112 side to the outer-tube tip end portion 111 side.

In the embodiment, the inner tube 120 has the tip-end-side projection 131 which is provided closer to the pushing tip end portion 121 than the protrusion 200 is. With this structure, the tip-end-side projection 131 comes in contact with the outer-tube tip end portion 111 (the opening 111A) after the protrusion 200 passes by the locking portion 213 from the outer-tube rear end portion 112 side to the outer-tube tip end portion 111 side. This further allows prevention of the inner tube 120 (the pushing tip end portion 121) from coming out from the outer tube 110.

In view of the above-described circumstances, it is preferable that the tip-end-side projection 131 be formed within 30 mm towards the pushing rear end portion 122 from the pushing tip end portion 121. If the tip-end-side projection 131 is formed in a position apart from the pushing tip end portion 121 by more than 30 mm, the inner tube 120 (the pushing tip end portion 121) comes out from the outer tube 110 in some cases.

In the embodiment, the inner tube 120 has the rear-end-side projection 132 which is provided closer to the pushing rear end portion 122 than the protrusion 200. With this structure, when the inner tube 120 pushes the tampon 10 toward the outside of the outer tube 110, the rear-end-side projection 132 comes in contact with the grip portion 212. This further allows prevention of the inner tube 120 from getting into the grip portion 212.

In view of the above-described circumstances, it is preferable that the rear-end-side projection 132 be formed within 30 mm towards the pushing tip end portion 121 from the pushing rear end portion 122. If the rear-end-side projection 132 is formed in a position apart from the pushing rear end portion 122 by more than 30 mm, the inner tube 120 cannot completely push the tampon 10 toward the outside of the outer tube 110 in some cases.

In the embodiment, it is preferable that the angular portion 205 at which the top face 203 and the front face 201 are continuous be formed in an arcuate shape (an R-letter shape). With this structure, it becomes easy for the protrusion 200 to get into the grip portion 212 and pass inside the grip portion 212. Accordingly, when the inner tube 120 pushes the tampon 10 toward the outside of the outer tube 110, the protrusion 200 can be securely prevented from being caught on the grip portion 212.

### (Variations of the Shape of the Protrusion)

The protrusion 200 according to the first embodiment has a substantially trapezoidal shape when seen from the side thereof. The shape of the protrusion 200 may be changed as follows. It should be noted that same reference signs are given to denote portions same as those of the protrusion 200 according to the first embodiment, and that the different portions are mainly described.

### (Variation 1)

A shape of a protrusion 210 according to Variation 1 is described by referring to the drawings. Fig. 6 (a) is a side view showing the protrusion 210 according to Variation 1. Fig. 6 (b) is a front view showing an inner tube 120 according to Variation 1.

As shown in Figs. 6 (a) and 6 (b), the protrusion 210 has a substantially triangular shape when seen from the side thereof and in an axial direction of the inner tube 120. A portion in which the protrusion 210 protrudes most is formed in an arcuate shape (an R-letter shape).

It should be noted that the portion where the protrusion 210 protrudes most does not necessarily have to have an arcuate shape, and may be pointed, for example. In addition, the protrusion 210 does not necessarily have to have a substantially triangular shape when seen from the side thereof and in the axial direction of the inner tube 120. The protrusion 210 may have a substantially triangular shape when seen either from the side thereof or in the axial direction of the inner tube 120.

### (Variation 2)

A shape of a protrusion 220 according to a second variation is described by referring to the drawings. Fig. 7(a) is a side view showing the protrusion 220 according to Variation 2. Fig. 7 (b) is a front view showing an inner tube 120 according to Variation 2.

As shown in Figs. 7 (a) and 7 (b), the protrusion 220 has a semi-circular shape when seen from the side thereof. It should be noted that the protrusion 220 does not necessarily have to have a semi-circular shape, and may have a semi-spherical shape or semi-elliptic shape, for example.

### (Variation 3)

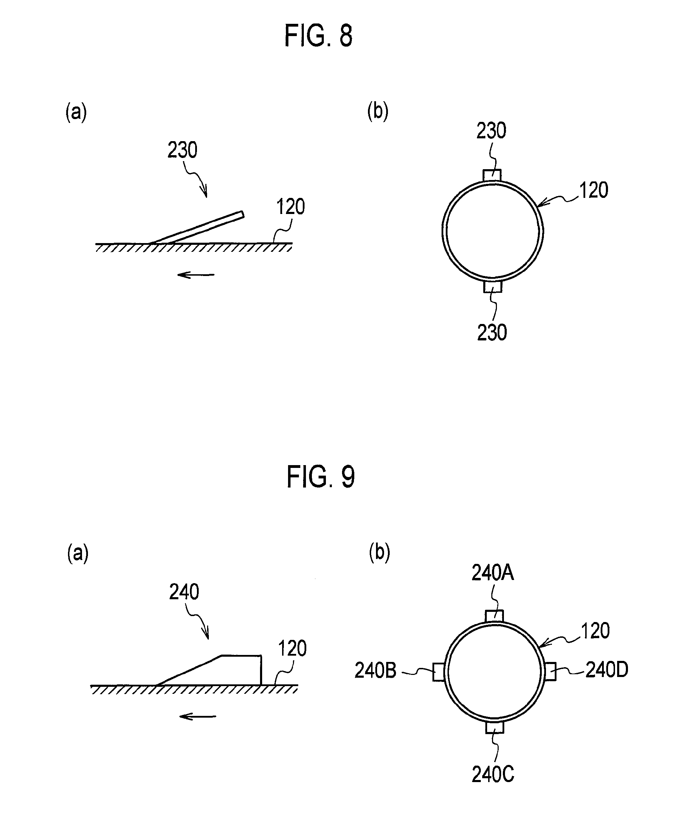

A shape of a protrusion 230 according to Variation 3 is described by referring to the drawings. Fig. 8 (a) is a side view showing the protrusion 230 according to Variation 3. Fig. 8 (b) is a front view showing an inner tube 120 according to Variation 3.

As shown in Figs. 8 (a) and 8 (b), the protrusion 230 has a plate shape including with respect to the outer circumference of the inner tube 120 extending toward the pushing rear end portion 122 while gradually rising outward from the outer circumference of the inner tube 120. The thickness of the protrusion 230 is substantially constant. It should be noted that the protrusion 230 does not necessarily have to have a constant thickness, and may have an uneven thickness.

The protrusion 230 may be provided on the outer circumference of the inner tube 120 as a separate body. Alternatively, the protrusion 230 may be formed by lifting a cutout formed in the inner tube 120.

### (Modifications in the Number and Layout of the Projections)

The protrusion 200 according to the first embodiment includes two protrusions 200 which are provided facing each other across the axis of the inner tube 120. The layout of the protrusions 200 may be modified as follows. It should be noted that the same reference sings are given to denote portions same as those of the protrusion 200 according to the first embodiment, and that different portions are mainly described.

### (Modification 1)

The number and layout of protrusions 240 according to Modification 1 are described by referring to the drawings. Fig. 9 (a) is a side view showing one of the protrusions 240 according to Modification 1. Fig. 9 (b) is a front view showing an inner tube 120 according to Modification 1.

As shown in Figs. 9 (a) and 9 (b), the protrusions 240 include four protrusions 240A to 240D which are provided facing each other across the axis of the inner tube 120. It should be noted that the number of the protrusions 240A to 240D does not necessarily have to be four, but may be four or more and be placed so as to be continuous over the entire region of the outer circumference of the inner tube 120.

### (Modification 2)

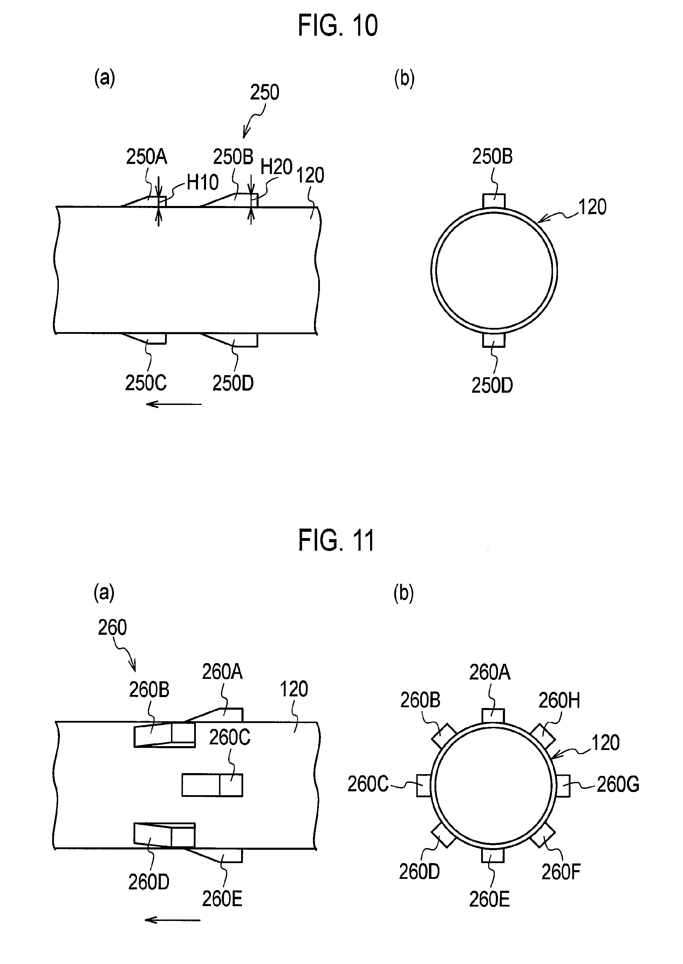

The number and layout of protrusions 250 according to Modification 2 are described by referring to the drawings. Fig. 10 (a) is a side view showing part of an inner tube 120 according to Modification 2. Fig. 10 (b) is a front view showing the inner tube 120 according to Modification 2.

As shown in Figs. 10 (a) and 10 (b), the protrusions 250 include four protrusions 250A to 250D. In the longitudinal direction of the inner tube 120, the protrusions 250A and 250B are provided in the same line, and the protrusions 250C and 250D are provided in the same line.

It is preferable that, of the four protrusions 250A to 250D, each of the protrusions 250A and 250C positioned closer to a pushing tip end portion 121 has a height H10 which is preferably smaller than a height H20 of each of the protrusions 250B and 250D. This is because such structure allows the protrusion 200 to easily get into the inside of the grip portion 212 and the inner tube 120 to be prevented from returning to the state before the sanitary tampon 1 is used.

### (Modification 3)

The number and layout of protrusions 260 according to Modification 3 are described by referring to the drawings. Fig. 11 (a) is a side view showing part of an inner tube 120 according to Modification 3. Fig. 11 (b) is a front view showing the inner tube according to Modification 3.

As shown in Figs. 11 (a) and 11 (b), the protrusions 260 include eight protrusions 260A to 260H. Each of the protrusions 260A to 260H is displaced from the adjacent protrusions in the longitudinal direction of the inner tube 120.

### [Second Embodiment]

Hereinafter, a tampon applicator 100A according to a second embodiment of the invention is described by referring to the drawings. It should be noted that the same reference signs are given to denote the same portions as those of the above-described tampon applicator 100 according to the first embodiment.

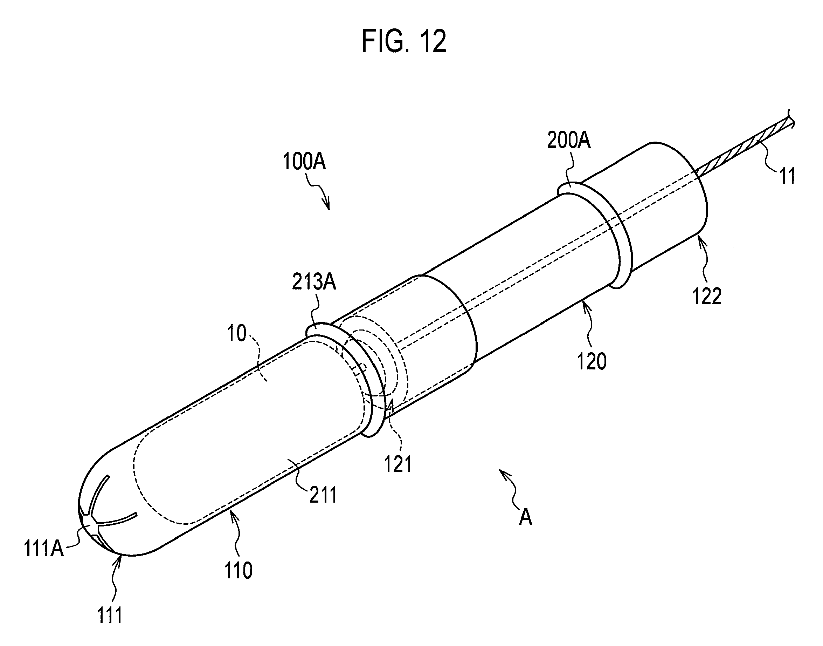

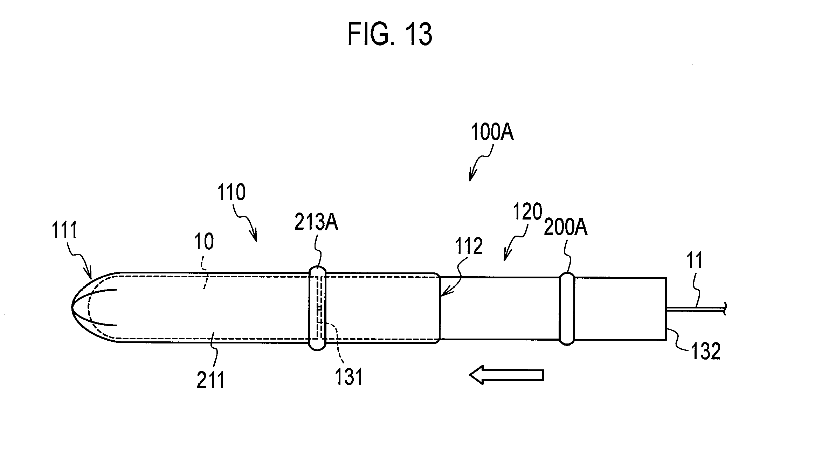

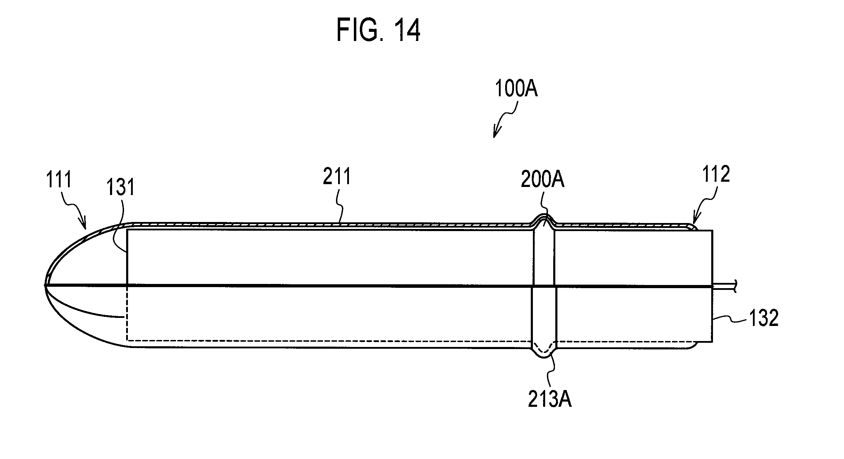

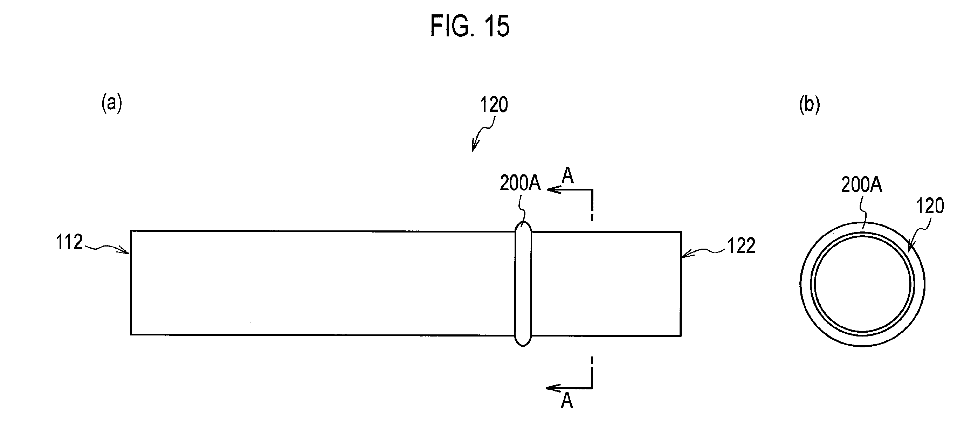

Fig. 12 is a perspective view showing a sanitary tampon applicator 100A according to the second embodiment. Fig. 13 is a side view (seen from the direction show by arrow A) showing a sanitary tampon 1A according to the second embodiment. Fig. 14 is a partially-exploded side view of an outer tube 110 according to the second embodiment. Fig. 15 (a) is a side view of an inner tube 120 according to the second embodiment. Fig. 15 (b) is a front view (a cross-sectional view taken along the A-A line in Fig. 15 (a)) showing the inner tube 120 according to the second embodiment.

In the first embodiment, the outer tube 110 includes the main body portion 211 and the grip portion 212. In contrast, in the second embodiment, the outer tube 110 has a constant diameter. It should be noted that the inner tube 120 has substantially the same diameter as the outer tube 110.

As shown in Figs. 12 to 15, the inner tube 120 is provided with a protrusion 200A formed continuously with an outer circumference of the inner tube 120. The outer tube 110 is provided with a locking portion 213A formed continuously with an outer circumference of the outer tube 110. The locking portion 213A projects in an inner circumferential surface of the outer tube 110 outward in a radial direction of the outer tube 110.

The protrusion 200A has the same shape as, or a shape smaller than, the locking portion 213A. It should be noted that various shapes described in the first embodiment (Variations 1 to 3 and Modifications 1 to 3) are applicable to the protrusion 200A and the locking portion 213A.

The protrusion 200A does not necessarily have to be formed continuously with the outer circumference of the inner tube 120. For example, the protrusion 200A may be at least one or more protrusions. In this case, it is preferable to consider that positions of the protrusion 200A and the locking portion 213A are displaced with each other in some cases.

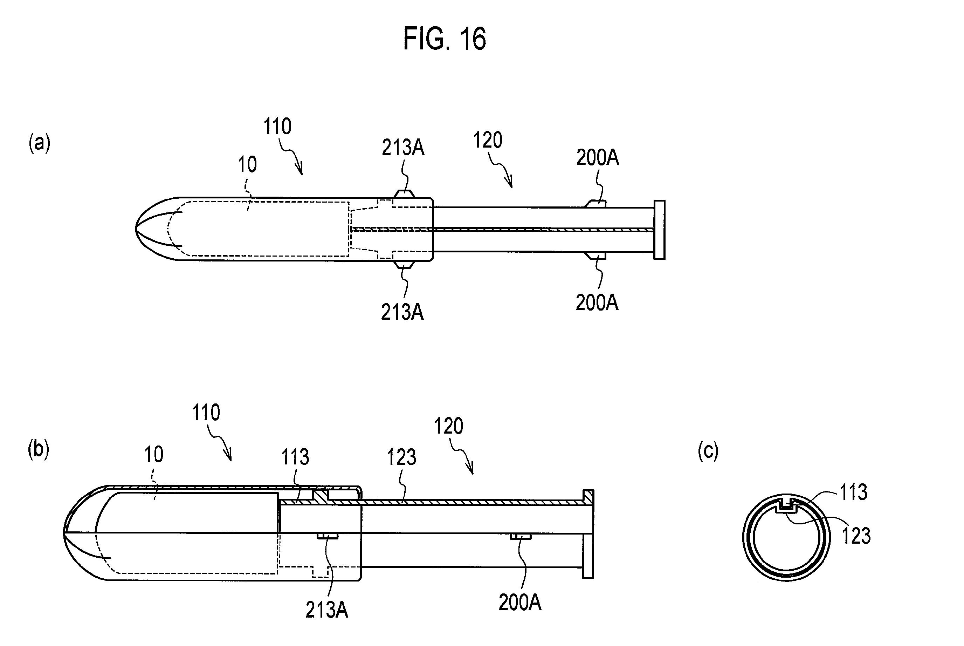

For example, the locking portion 213A may be formed continuously with the outer circumference of the outer tube 110. In addition, as shown in Fig. 16, the inner tube 120 may have a rail 123 recessed in an inner circumference thereof, and the outer tube 110 may have a rail 113 protruding from an inner circumference thereof. It should be noted that the rail 123 may be formed in a projection shape, in which case the rail 113 is formed in a recess shape.

In the above-described second embodiment, similar to the advantageous effects of the first embodiment, after the protrusion 200A passes by the locking portion 213A from the outer-tube rear end portion 112 side to the outer-tube tip end portion 111 side, the locking portion 213A locks the protrusion 200A. As a result, the inner tube 120 does not return to the state before the sanitary tampon 1A is used. This surely prevents the user from accidentally using the sanitary tampon 1A in which the tampon 10 is not placed in the tampon applicator 100A.

### [Other Embodiments]

As described above, the details of the present invention have been disclosed by using the embodiments of the present invention. However, it should not be understood that the description and drawings which constitute part of this disclosure limit the present invention. From this disclosure, various alternative embodiments, examples, and operation techniques will be easily found by those skilled in the art.

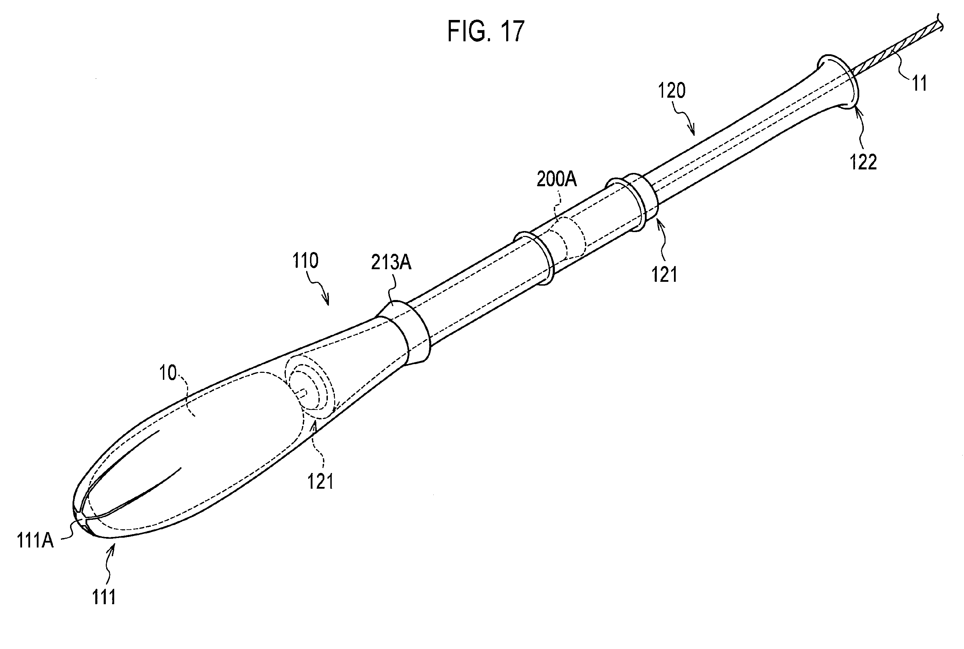

For example, the embodiments of the invention can be modified as follows. Specifically the sanitary tampon 1 is not limited to the sanitary tampons 1 described in the first and second embodiments, but is applicable for example to a shape of the sanitary tampon 1, i.e., shapes of an outer tube 110 and an inner tube 120, as shown in Fig. 17, . In this case, a locking portion 213A formed in the outer tube 110 protrudes in an inner circumferential surface of the outer tube 110 outward in the radial direction of the outer tube 110. In addition, it is assumed that the protrusion 200A has the same shape as, or a shape smaller than, the locking portion 213A.

Moreover, the outer tube 110 and the inner tube 120 have been described as being formed of the same material. However, the present invention is not limited to such case, and the outer tube 110 may be formed of a material softer than the inner tube 120 so that the protrusion 200 can easily pass inside the grip portion 212.

The thicknesses of the outer tube 110 and the inner tube 120 are not particularly limited. For example, the thickness of the outer tube 110 may be smaller than the thickness of the inner tube 120 so that the protrusion 200 can easily pass inside the grip portion 212.

Moreover, the inner tube 120 has been described as being a hollow tube. However, the inner tube 120 is not limited to this, and may of course have a different shape, such as a prismatic shape, as long as it can push the tampon 10 to the outside of the outer tube 110 and as long as a cord-like body 11 can be inserted therein.

The shape, structure, number, and layout of the protrusion 200 are not particularly limited and may be any ones other than those described in the above-described embodiments and may be selectable appropriately.

As described above, the present invention naturally includes various embodiments which are not described herein. Accordingly, the technical scope of the present invention should be determined only by the matters to define the invention in the scope of claims regarded as appropriate based on the description.

Note that the entire content of Japanese Patent Application No. 2009-061700 (filed on March 13, 2009) is incorporated by reference into this application.

### [Industrial Applicability]

Since the present invention can provide a tampon applicator capable of securely preventing a user from accidentally using a sanitary tampon in which a tampon is not placed in the tampon applicator, it is useful in manufacturing of the tampon applicator.

### [Reference Signs List]

1, 1A: sanitary tampon, 10: tampon, 11: cord-like body, 100, 100A: tampon applicator, 110: outer tube, 111: outer-tube tip end portion, 111A: opening, 112: outer-tube rear end portion, 113: rail, 120: inner tube (pushing body), 121: pushing tip end portion, 122: pushing rear end portion, 123: rail, 131: tip-end-side projection, 132: rear-end-side projection, 200, 200A (210,220,230,240,250,260): protrusion, 201: front face (front portion), 202: rear face (rear portion), 203: top face, 204: side face, 211: main body portion, 212: grip portion, 213, 213A: locking portion

## Claims

1. A tampon applicator comprising:
an outer tube in which a tampon is ejectably placed; and
a pushing body which pushes the tampon toward the outside of the outer tube by slidingly moving in the outer tube, wherein
the outer tube includes:
an outer-tube tip end portion, on which an opening to eject the tampon therefrom is formed, and which is inserted into the vagina of a user;
and an outer-tube rear end portion positioned opposite to the outer-tube tip end portion,
the pushing body includes:
a pushing tip end portion pushing the tampon toward the outside of the outer tube;
a pushing rear end portion positioned opposite to the pushing tip end portion; and
a protrusion which is provided in the vicinity of the pushing rear end portion and protrudes from the outer circumference of the pushing body,
the outer tube is provided with a locking portion which locks the protrusion when the pushing body is inserted in the outer tube,
along with a sliding movement of the pushing body in the outer tube, the protrusion is capable of passing by the locking portion from the outer-tube rear end portion side to the outer-tube tip end portion side, and
when the protrusion is positioned on the outer-tube tip end portion side of the locking portion, the locking portion locks the protrusion to prevent the pushing body from returning to the outer-tube rear end portion side of the locking portion.

2. The tampon applicator according to claim 1, wherein
the outer tube includes:
a main body portion having a predetermined diameter; and
a small diameter portion which is provided closer to the outer-tube rear end portion than the main body portion is and which has a diameter smaller than the diameter of the main body portion, and
the locking portion is provided between the main body portion and the small diameter portion.

3. The tampon applicator according to claim 1, wherein
a diameter of the outer tube is constant,
the locking portion protrudes in an inner circumferential surface of the outer tube outward in a radial direction of the outer tube, and
the protrusion has a shape the same as the locking portion or a shape smaller than the locking portion.

4. The tampon applicator according to any one of claims 1 to 3, wherein the outer tube is formed of a material softer than the material of the pushing body

5. The tampon applicator according to any one of claims 1 to 4, wherein
the pushing body has a tubular shape, and
a thickness of the outer tube is smaller than a thickness of the pushing body.

6. The tampon applicator according to any one of claims 1 to 5, wherein
the protrusion has a front portion facing toward the pushing tip end portion, and
the front face is inclined with respect to the outer circumference of the pushing body and extends toward the pushing rear end portion while rising outward from the outer circumference of the pushing body

7. The tampon applicator according to claim 6, wherein
the protrusion has a rear portion opposite to the front face and is positioned closer to the pushing rear end portion than the front portion, and
the rear portion is substantially perpendicular to the outer circumference of the pushing body

8. The tampon applicator according to claim 2, wherein a length from the protrusion to the pushing rear end portion in a longitudinal direction of the pushing body is larger than a length of the small diameter portion in a longitudinal direction of the outer tube.

9. The tampon applicator according to claim 2, wherein a length from the protrusion to the pushing tip end portion in a longitudinal direction of the pushing body is smaller than a length of the main body portion in a longitudinal direction of the outer tube.

10. The tampon applicator according to claim 2, wherein the pushing body includes:
a tip-end-side projection which is provided closer to the pushing tip end portion than the protrusion and
projects from the outer circumference of the pushing body outward beyond the diameter of the small diameter portion; and
a rear-end-side projection which is provided closer to the pushing rear end portion than the protrusion is and
projects from the outer circumference of the pushing body outward beyond the diameter of the small diameter portion.
